# EUROPÄISCHE PATENTSCHRIFT

(11) **EP 0 989 881 B1**
(45) Veröffentlichungstag und Bekanntmachung des Hinweises auf die Patenterteilung: **03.08.2005**
(21) Anmeldenummer: 98934823.0
(22) Anmeldetag: 28.05.1998
(51) Int. Cl.: A61M 39/06, A61M 39/28

(54) **KATHETEREINFÜHRVORRICHTUNG MIT EINER EINRICHTUNG ZUM FLÜSSIGKEITSDICHTEN ABKLEMMEN DES EINFÜHRUNGSLUMENS**
CATHETER INSERTION DEVICE WITH A SYSTEM FOR LIQUID-TIGHT CLAMPING OF THE INSERTION LUMEN
DISPOSITIF DE GUIDAGE DE CATHETER MUNI D'UN SYSTEME PERMETTANT D'OBTURER LE LUMEN D'INTRODUCTION PAR PINCEMENT, DE MANIERE ETANCHE AUX LIQUIDES

(30) Priorität: 31.05.1997 DE 19722753
(43) Veröffentlichungstag der Anmeldung: 05.04.2000
(73) Patentinhaber: Von Casimir, Wolf, 82340 Feldafing (DE)
(72) Erfinder: Von Casimir, Wolf, 82340 Feldafing (DE)
(74) Vertreter: Czybulka, Uwe, Dipl.-Phys.
(86) Internationale Anmeldenummer: PCT/DE1998/001466
(87) Internationale Veröffentlichungsnummer: WO 1998/053874

(56) Entgegenhaltungen:
- EP-A- 0 455 478
- EP-A- 0 546 712
- DE-C- 3 324 699
- US-A- 5 158 553

## Beschreibung

Die Erfindung betrifft ein Einführsystem für den Einsatz bei Eingriffen in das Lumen eines Blutgefäßes, zum Einführen und Auswechseln von Behandlungsinstrumentengemäß dem Patentanspruch 1.

Zur Einführung von Kathetern in Blutgefäße bedient man sich üblicherweise der Seldinger-Technik. Hierdurch wird ein aus der Körperoberfläche herausragender, "Hämostatischer Einführer" genannter Zugang gelegt, dessen zentrale Öffnung mittels eines sternförmig geschlitzten Stopfens aus weichelastischem Material zunächst selbsttätig geschlossen ist. Durch diese Öffnung wird sodann ein Führungskatheter mit seinem vorderen Ende in das Lumen des Blutgefäßes so weit vorgeschoben, bis die Spitze des Führungskatheters möglichst nahe vor den mit den eigentlichen Behandlungskathetern beziehungsweise -instrumenten zu erreichenden Behandlungsort gelangt. Das rückwärtige Führungskatheterende liegt dann außerhalb der Öffnung des Hämostatischen Einführers. An diesem Führungskatheterende ist ein als "Einführventil" bezeichneter, beispielsweise mittels eines Schraubverschlusses mit einer zentralen Öffnung zu schließender und teilweise oder ganz zu öffnender Zugang angeschlossen.

Das Einführventil ist nach dem Stopfbuchsenprinzip aufgebaut und weist einen weichelastischen, zylindrischen Dichtring mit einer zentralen Öffnung auf. Dieser Dichtring wird, je nach der gewünschten Dichtwirkung beziehungsweise einzustellenden Restöffnung, mit Hilfe eines achsial verschiebbaren Ringkolbens durch Zusammenpressen in seiner Form angepasst.

Um nun, beispielsweise zur Durchführung einer Koronargefäß-Dilatation mittels eines Ballondilatationskatheters, einen Führungsdraht und anschließend den Behandlungskatheter mit der jeweiligen, verhältnismäßig empfindlichen Spitze unbeschädigt in das Lumen des Blutgefäßes einzubringen und bis zu der zu behandelnden Stelle vorzuschieben, muss die Zugangsöffnung des Einführventils in der Regel vollständig geöffnet werden. Während der Zeitdauer, bis sie wieder so weit geschlossen werden kann, dass der Führungsdraht beziehungsweise der Behandlungskatheter dicht umschlossen und gleichzeitig in Längsrichtung verschiebbar ist, tritt zwangsläufig eine mehrere Milliliter umfassende Blutmenge aus.

Wie bei der zuvor beschriebenen Einführung des Führungsdrahtes und des Behandlungskatheters, wiederholen sich im wesentlichen diese Einzelheiten einschließlich des Blutaustretens bei sämtlichen weiteren Maßnahmen zur Auswechslung der Katheter, da das Einführventil in jedem Fall zumindest einige Sekunden lang geöffnet werden muss. Der sich im Zuge einer derartigen Behandlung auf diese Weise ergebende Gesamtblutverlust kann, insbesondere bei Patienten mit Bluthochdruck, durchaus erhebliche Maße annehmen. Die Verunreinigung der Umgebung des Einführventils durch Blut wird bislang als eine unvermeidbare Belästigung hingenommen. Von den Patienten kann das Blutaustreten, sofern sie es wahrnehmen, möglicherweise als psychisch belastend empfunden werden.

Die Erfindung löst die Aufgabe, herkömmliche Einführsysteme dahingehend zu verbessern beziehungsweise zu ergänzen, dass bei ihrem Benutzen jegliches Austreten von Blut vermeidbar ist.

Diese Aufgabe wird durch die Merkmale des Anspruches 1 gelöst.

Die absperrbare Einführöffnung des Einführventiles mit ihrem Dichtring und das Sperrelement erfüllen die Funktion quasi zweier Schleusentore an den Enden einer Schleusenkammer in dem Kanal, der sich vom Dichtring bis in das Lumen des Führungskatheters hinein erstreckt. Diese Funktionen vermögen der Dichtring und das Sperrelement auch zu übernehmen, wenn ein Führungsdraht im Kanal liegt.

Das erfindungswesentliche Sperrelement ermöglicht, sofern es in die Schließstellung gebracht wird, das beliebige Öffnen des Einführventils, ohne dass Blut aus dessen zentraler Einführöffnung austritt. Sobald dann anschließend als erstes der Führungsdraht, später der Katheterkopf, beispielsweise im Falle eines Ballondilatationskatheters dessen Spitze und der Dilatationsballon und zumindest ein kurzes Stück des unmittelbar anschließenden Katheterschaftes, durch den geöffneten Dichtring des Einführventils geschoben sind, können der Dichtring und damit dessen zentrale Einführöffnung, ohne Zwang zu eiliger Betätigung, so an den Durchmesser des Führungsdrahtes oder des Katheterschaftes angepasst werden, dass einerseits die notwendige Dichtwirkung erzielt wird und andererseits der Führungsdraht beziehungsweise der Katheterschaft in Längsrichtung zu bewegen sind. Gegebenenfalls wird der vor der Einführung des Katheters bereits bis zur Behandlungsstelle gelegte Führungsdraht vom geschlossenen Sperrelement festgehalten und somit gegen ein Längsverschieben gesichert. Dadurch kann der Kopf eines folgenden Behandlungskatheters vom außerhalb liegenden Führungsdrahtende her, ohne dass dieses gesondert festhalten werden muss, auf den Führungsdraht aufgeschoben werden. Nach diesen Schritten kann das Sperrelement geöffnet und der Katheterkopf in Richtung der Behandlungsstelle verlegt werden.

Aus der DE-C1-33 24 699 ist zum Ausführen einer zentralen venösen Punktion eine in einem Punktionsbesteck angeordnete Ventilvorrichtung in Gestalt eines Schlauchstückes bekannt, dessen Innenquerschnitt durch Verdrillen geschlossen werden kann.

Aus der EP-A1-0 455 478 ist eine Ventilvorrichtung für einen sogenannten "over-the-needle-in"-Katheter, d. h. einen Kurzkatheter bekannt, der mittels einer mit der Spitze über sein distales Ende hinausragenden Punktionsnadel als Zugang zu einem Blutgefäß zu legen ist. Der Katheter weist proximal zu dem Katheterkanal ein Schlauchstück auf, das mit einem hakenartigen Hebel von außen zusammen gequetscht werden kann, sodass das Schlauchstück flüssigkeitsdicht abgesperrt werden kann.

Das Einführsystem mit der Schleusenkammer kann an einer geeigneten Stelle, vorzugsweise im Bereich des Sperrelementes, einen in den Hauptkanal einmündenden Nebenkanal aufweisen, durch den eine sterile, durchsichtige Flüssigkeit, günstigerweise physiologische Kochsalzlösung, in das Lumen des Hauptkanals zwischen der Einmündungsstelle und dem Dichtring des Einführventils eingebracht werden kann. Die Verwendung eines durchsichtigen Wandmaterials bei der Herstellung zumindest des Hauptkanals zwischen dem Sperrelement und dem Dichtring ermöglicht es, die Lage eines jeden Katheterkopfes beim Einführvorgang zu beobachten. Im übrigen stellt die Flüssigkeit, die in gleicher Weise am Austreten aus dem Einführventil gehindert ist wie ansonsten das Blut, einen "sauberen Zusatzverschluss" des Systems dar.

Zudem ist ein weiterer zusätzlicher Nebenkanal möglich, beispielsweise möglichst nahe am Dichtring, über den die Flüssigkeit ohne ein auch nur teilweises Öffnen des Einführventils eingefüllt oder ausgetauscht werden kann, falls sie sich infolge des Längsverschiebens der Behandlungskatheter allmählich mit Blut vermischen sollte.

Im folgenden werden vorteilhafte Ausführungsformen der Erfindung anhand der Figuren in der Zeichnung näher erläutert:
- Figur 1a und 1b: zeigen schematisch ein Sperrelement in geschlossener und in geöffneter Stellung,
- Figur 2: enthält die Darstellung eines Sperrelementes mit an seinem Eingang und seinem Ausgang angeordneten, genormten Steckanschlüssen, in diesem Fall Luer-Anschlüssen,
- Figuren 3a, 3b und 3c: zeigen die wesentlichen Anordnungsweisen des erfindungsgemäßen Sperrelementes im jeweiligen Einführsystem.

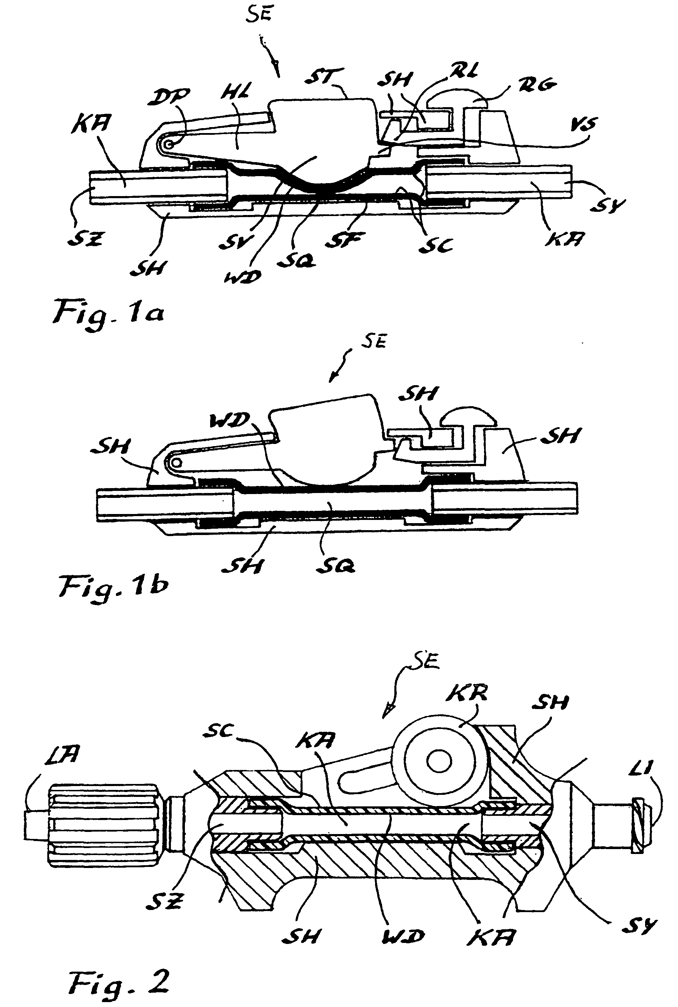

Das in den Figuren 1a und 1b dargestellte Sperrelement SE ist nach dem Prinzip einer Schlauchklemme aufgebaut. Die beiden wesentlichsten Teile des Sperrelementes SE sind das Schlauchstück SC, durch das der sich vom Einführventil EV bis in den Führungskatheter erstreckende Kanal KA verläuft, und die Stellvorrichtung SV. Das Schlauchstück SC liegt im Wirkungsbereich der Stellvorrichtung SV auf der Stützfläche SF auf, während die Stellvorrichtung SV an einem Hebel HL befestigt ist, der sich um den Drehpunkt DP schwenkbar am Sperrelementgehäuse SH abstützt. Mittels der Sperrtaste ST kann die Stellvorrichtung SV gegen das Schlauchstück SC gedrückt werden, bis dessen Innenquerschnitt (Sperrquerschnitt SQ) flüssigkeitsdicht verschlossen ist. In dieser Schließstellung wird die Stellvorrichtung SV durch einen Riegel RL gehalten, der in den Schwenkbereich eines Vorsprunges VS am Hebel HL ragt.. Wird der Riegel RL mittels des Riegelgriffes RG vom Vorsprung VS fortbewegt, springt die Stellvorrichtung SV - bewirkt durch den Druck im Schlauchstück SC, dessen Wandung WD sich durch den Druck zurückverformt - in die in Figur 1b gezeigte Offenstellung des Sperrelementes SE.

Der Riegel RL wird in bekannter Weise durch ein Federelement, das nicht eigens dargestellt ist, in der Stellung gehalten, die der Schließstellung entspricht. Der Riegel RL ist darüberhinaus mit einer geeignet ausgebildeten Schrägfläche versehen, die in Verbindung mit einer entsprechend gestalteten Gegenfläche am Vorsprung VS ein Bewegen der Stellvorrichtung SV, unter Verschieben des Riegels RL, in die Schließstellung ermöglicht. Ist diese Stellung erreicht, wird der Riegel RL von dem Federelement in die Schließstellung zurückgedrückt. Das Schlauchstück SC des Sperrelementes SE geht an seinen beiden Enden in den Sperrelementeingang SY beziehungsweise in den Sperrelementausgang SZ des Sperrelementgehäuses SH über. Die bei SY gelegene Seite entspricht der Einführseite eines Führungskatheters und wird als proximale Seite des Sperrelementes bezeichnet. Die zum Blutgefäß zeigende Seite bei SZ ist die distal gelegene Seite des Sperrelementes.

Figur 2 zeigt eine Ausführungsform des Sperrelementes SE mit zwei jeweils am Sperrelementeingang SY und am Sperrelementausgang SZ angeordneten Luer-Anschlüssen, in dem dargestellten Beispiel einem Luer-Innenkegel LI am Sperrelementeingang SY und einem Luer-Außenkegel LA mit einer Überwurfsicherung am Sperrelementausgang SZ. Dieses Sperrelement SE ist auf einfache Weise zwischen herkömmliche Einführventile und Führungskatheter einfügbar und kann in einfacher Spritzgusstechnik hergestellt werden. Das Einführventil ist mit seinem Anschlussstück an den Luer-Innenkegel LI anschließbar, der Führungskatheter mit seinem Anschlussstück entsprechend an dem Luer-Außenkegel LA.

Als Stellvorrichtung SV weist das Sperrelement SE eine Klemmroll.e KR auf, die mit ihrer Achse in einer im Winkel zur Achse des Schlauchstückes SC angeordneten Rillenführung gerollt werden kann und in der jeweils gewählten Stellung durch Reibung gehalten wird. Diese Ausführungsform ähnelt den bekannten Rollenklemmen, die sich zur Drosselung oder zum Verschließen von Infusionsleitungen bewährt haben.

Andere Möglichkeiten zur Verwirklichung eines erfindungsgemäßen Sperrelementes SE bestehen beispielsweise in einem Spannzangenmechanismus oder in einer Anordnung, bei der ein um das Schlauchstück SC in der Ebene des Sperrquerschnittes SQ außen um die Schlauchwandung gelegtes Band durch Auseinanderziehen seiner Enden das Schlauchstück SC abschnüren kann. Derartige, hier nur allgemein angesprochene Ausführungsformen stellen ebenfalls Verwirklichungen des Erfindungsgedankens dar, wie auch sämtliche äquivalenten Ausgestaltungen.

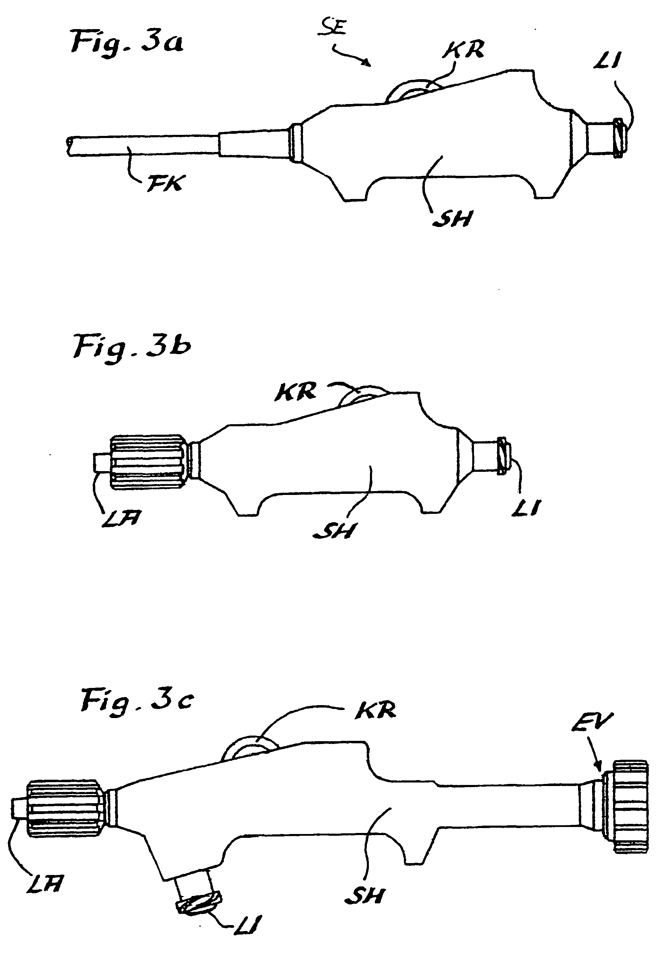

Die Figuren 3a bis 3c enthalten schematische Darstellungen der drei wesentlichen erfindungsgemäßen Einsatzarten des Sperrelementes SE, in denen es seine Funktion zur Lösung der gestellten Aufgabe erfüllen kann. Wie in Figur 3a gezeigt, kann es beispielsweise in den Schaft des Führungskatheters FK eingefügt sein. An dem proximalen Ende ist noch ein Luer-Anschluss LI vorgesehen, an den z. B. das Einführventil anschließbar ist.

Die zweite Möglichkeit, das Sperrelement SE als ein mittels Luer-Anschlüssen zwischen ein Einführventil EV und einen Führungskatheter FK einzukoppelndes Zusatzteil zum herkömmlichen Einführinstrumentarium auszuführen, ist bereits oben beschrieben; die entsprechende Darstellung hierzu findet sich in Figur 3b.

Die dritte sinnvolle Ausführungsmöglichkeit ist in Figur 3c wiedergegeben: in diesem Fall ist das Sperrelement SE im Einführventilschaft des Einführventils EV angeordnet. Dabei ist es günstig, wenn der Abstand zwischen dem Dichtring des Einführventils EV und dem Schlauchstück SC des Sperrelementes SE größer als die Länge des Kopfteiles des einzuführenden Behandlungskatheters ist; unter "Kopfteil" ist hier derjenige Abschnitt des Behandlungskatheters zu verstehen, der sich von der Katheterspitze bis zum Übergang in den Hauptschaft des Katheters, d.h. in den nächstliegenden Bereich gleichbleibender Querschnittsaußenform des Hauptschaftes erstreckt. In Figur 3c ist auch ein Nebenkanal mit einem Lumen LI gezeigt, in den z. B. eine Spritze eingeführt werden kann, um eine sterile durchsichtige Spülflüssigkeit in die Schleusenkammer einzubringen.

Der Dichtring des Einführventils EV und das Sperrelement SE erfüllen die Funktion quasi zweier Schleusentore an den Enden einer Schleusenkammer in dem Kanal KA. Diese Funktionen vermögen der Dichtring und das Sperrelement SE auch zu übernehmen, wenn ein Führungsdraht im Kanal KA liegt.

## Patentansprüche

1. Einführsystem für den Einsatz bei Eingriffen in das Lumen eines Blutgefäßes, zum Einführen und Auswechseln von Behandlungsinstrumenten, mit folgenden Merkmalen:
- einem Führungskatheter (FK) mit einem Führungskatheterlumen (FL) und einem mit dem Führungskatheter (FK) verbundenen Einführventil (EV) mit einer einen Dichtring aufweisenden und durch diesen absperrbaren Eingangsöffnung ;
- einem sich von der Eingangsöffnung des Einführventiles (EV) bis in das Führungskatheterlumen (FL) erstreckenden Kanal (KA) mit einem im wesentlichen gleich bleibenden Kanalinnenquerschnitt (KQ), wobei an einer ausgewählten Stelle des Kanales (KA) zwischen dem Einführventil und dem Führungskatheterlumen (FL) ein Sperrelement (SE) eingefügt ist, in dem ein rohrartiger Kanalabschnitt (SC) mit einer umkehrbar verformbaren Wandung (WD) angeordnet ist, die mittels einer von außen einwirkenden Stellvorrichtung (SV) dergestalt verformbar ist, dass der Kanalinnenquerschnitt an dieser Stelle (SQ) flüssigkeitsdicht absperrbar ist;
- wobei zwischen dem Dichtring des Einführventiles (EV) und dem Sperrelement (SE) in dem Kanal eine Schleusenkammer ausgebildet ist.

2. Einführsystem nach Anspruch 1, **dadurch gekennzeichnet, dass** der rohrartige Kanalabschnitt ein rohrartiges Schlauchstück (SC) ist .

3. Einführsystem nach Anspruch 2, **dadurch gekennzeichnet, dass** das Schlauchstück einen im Wesentlichen gleich bleibenden Innenquerschnitt aufweist.

4. Einführsystem nach einem der vorhergehenden Ansprüche, **dadurch gekennzeichnet, daß** das Sperrelement (SE) ein separates Teil ist, das zwischen dem proximalen Ende des Führungskatheters (FK) und dem distalen Ausgang des Einführventils (EV) einfügbar ist.

5. Einführsystem nach Anspruch 4, **dadurch gekennzeichnet, dass** das Sperrelement (SE) eigene Anschlussstücke (LA, LI) aufweist, über die das Sperrelement mit korrespondierenden Anschlussstücken des Einführventiles (EV) bzw. des Führungskatheters (FK) verbindbar ist.

6. Einführsystem nach einem der vorhergehenden Ansprüche, **dadurch gekennzeichnet, daß** das Sperrelement (SE) in einem gesonderten Sperrelementgehäuse (SH) gehalten ist.

7. Einführsystem nach Anspruche 1, **dadurch gekennzeichnet, dass** das Sperrelement (SE) in den Führungskatheterschaft in einem nahen Abstand zum Führungskathetereingang am proximalen Ende des Führungskatheter eingefügt ist.

8. Einführsystem nach einem der vorhergehenden Ansprüche, **dadurch gekennzeichnet, dass** das Sperrelement (SE) mit dem distalen Ende des Einführventiles (EV) fest verbunden bzw. mit diesem einstückig ist.

## Claims

1. An insertion system for use when entering the lumen of a blood vessel, for inserting and changing treatment instruments, comprising the following features:
- a guide catheter (FK) having a guide catheter lumen (FL) and an insertion valve (EV) connected to the guide catheter (FK), which comprises an inlet opening having a sealing ring which can be shutoff by means of said sealing ring;
- a channel (KA) extending from the inlet opening of the insertion valve (EV) into the guide catheter lumen (FL), which comprises a substantially constant inner channel cross-section (KQ), a shutoff element (SE) being inserted at a selected location of the channel between the insertion valve and the guide catheter lumen (FL), in which a tubular channel portion (SC) with a reversibly deformable wall (WD) is disposed, which can be deformed by means of a control device (SV) acting from the outside in such a way that the inner channel cross-section can be shut off in a fluid-tight fashion at this location (SQ);
- a lock chamber being formed in the channel between the sealing ring of the insertion valve (EV) and the shutoff element (SE).

2. An insertion system according to claim 1, **characterized in that** the pipe-shaped channel section is a pipe-shaped hose piece.

3. An insertion system according to claim 2, **characterized in that** the hose piece has a substantially constant inner cross-section.

4. An insertion system according to any of the preceding claims, **characterized in that** the shutoff element (SE) is a separate part which can be inserted between the proximal end of the guide catheter (FK) and the distal outlet of the insertion valve (EV).

5. An insertion system according to claim 4, **characterized in that** the shutoff element (SE) comprises attachments (LA, LI) of its own by means of which the shutoff element can be connected with corresponding attachments of the insertion valve (EV) and/or the guide catheter (FK).

6. An insertion system according to any of the preceding claims, **characterized in that** the shutoff element (SE) is held in a separate shutoff element housing (SH).

7. An insertion system according to claim 1, **characterized in that** the shutoff element (SE) is inserted in the guide catheter shaft at a close distance to the guide catheter inlet at the proximal end of the guide catheter.

8. An insertion system according to any of the preceding claims, **characterized in that** the shutoff element (SE) is firmly connected to the distal end of the insertion valve (EV) and/or forms one piece with it.

## Revendications

1. Système d'introduction destiné à être utilisé lors d'interventions dans le lumen d'un vaisseau sanguin, pour introduire et échanger des instruments de soins, présentant les caractéristiques suivantes :
- un cathéter de guidage (FK) comportant un lumen de cathéter de guidage (FL) et une soupape d'introduction (EV) reliée au cathéter de guidage (FK) avec un orifice d'entrée présentant une bague d'étanchéité et susceptible d'être obturé par celle-ci ;
- un canal (KA) s'étendant depuis l'orifice d'entrée de la soupape d'introduction (EV) jusque dans le lumen de cathéter de guidage (FL) avec une section transversale intérieure de canal (KQ) restant sensiblement constante, entre la soupape d'introduction et le lumen de cathéter de guidage (FL) étant inséré un élément de blocage (SE) dans lequel est agencé un tronçon de canal (SC) tubulaire avec une paroi (WS) réversiblement déformable qui peut être déformée au moyen d'un dispositif de réglage (SV) agissant depuis l'extérieur de telle sorte que la section transversale intérieure de canal peut être obturée de manière étanche aux liquides à cet endroit (SQ),
- un sas étant réalisé dans le canal entre la bague d'étanchéité de la soupape d'introduction (EV) et l'élément de blocage (SE).

2. Système d'introduction selon la revendication 1, **caractérisé en ce que** le tronçon de canal tubulaire est un morceau de tuyau tubulaire (SC).

3. Système d'introduction selon la revendication 2, **caractérisé en ce que** le morceau de tuyau présente une section transversale intérieure qui reste sensiblement constante.

4. Système d'introduction selon l'une des revendications précédentes, **caractérisé en ce que** l'élément de blocage (SE) est une pièce séparée qui peut être insérée entre l'extrémité proximale du cathéter de guidage (FK) et la sortie distale de la soupape d'introduction (EV).

5. Système d'introduction selon la revendication 4, **caractérisé en ce que** l'élément de blocage (SE) présente des pièces de raccordement (LA, LI) propres par l'intermédiaire desquelles l'élément de blocage peut être relié avec des pièces de raccordement correspondantes de la soupape d'introduction et du cathéter de guidage (FK), respectivement.

6. Système d'introduction selon l'une des revendications précédentes, **caractérisé en ce que** l'élément de blocage (SE) est maintenu dans un boîtier d'élément de blocage (SH) séparé.

7. Système d'introduction selon la revendication 1, **caractérisé en ce que** l'élément de blocage (SE) est inséré dans le fût de cathéter de guidage à une distance proche de l'entrée du cathéter de guidage, à l'extrémité proximale du cathéter de guidage.

8. Système d'introduction selon l'une des revendications précédentes, **caractérisé en ce que** l'élément de blocage (SE) est relié de manière solidaire à l'extrémité distale de la soupape d'introduction (EV) ou est d'un seul tenant avec celle-ci.
